# EUROPEAN PATENT APPLICATION

(11) **EP 3 156 039 A1**
(43) Date of publication of application: **19.04.2017**
(21) Application number: 15806066.5
(22) Date of filing: 14.04.2015
(51) Int. Cl.: A61K 8/63, A61K 31/47, A61K 31/56

(54) **SPECIFIC MODULATORS OF CONNEXIN HEMICHANNELS**

(30) Priority: 12.06.2014 CL 20141556
(71) Applicant: Pontificia Universidad Católica de Chile, Santiago (CL)
(72) Inventor: SAEZ, Juan Carlos, Santiago (CL); LAGOS, Carlos, Santiago (CL)
(74) Representative: Illescas, Manuel
(86) International application number: PCT/CL2015/050012
(87) International publication number: WO 2015/188288

(57) **Abstract**

The invention provides identification methods of specific modulators of hemichannels formed by connexin through the use of structural bioinformation methods for the calculation of the interaction energy with ligands and compositions to selectively modulate the activity of hemichannels formed by connexins.

Among other aspects, the invention describes a computer-aided method, where estimating the interaction energy between ligands coming from a database of chemical compounds is possible, with a binding pocket defined by the region comprising the residues of 3-10 (NTH), 29-40 (TM1), 74-93 (TM2) of a protomer and residues 29-40 (TM1) of an adjacent protomer in connexin 26 or the equivalent residues in other connexins.

In particular, the invention provides compounds specifically inhibiting the activation/opening of connexin hemichannels identified through the method described above, which can be useful for the treatment of inflammatory diseases, vascular disorders, arrhythmias, chronic injuries, retinal neuroprotection, treatment of pain, skeletal muscle denervation, muscular dystrophies, damage to the spinal cord and genetic diseases characterized by the increased activity of hemichannels formed by connexins.

## Description

### FIELD OF THE INVENTION

The present invention refers to methods to identify specific modulating compounds (blockers) of hemichannels formed by connexins, the new use of the compounds identified under this methodology and the pharmaceutical compositions comprising those compounds to treat diseases associated with the increased activity of hemichannels formed by connexins, such as inflammatory diseases, vascular disorders, arrhythmias, chronic injuries, retinal neuroprotection, treatment of pain, skeletal muscle denervation, muscular dystrophies, post-ischemia reperfusion (example: cardiac or cerebral infarction), damage to the spinal cord and genetic diseases characterized by the increased activity of hemichannels formed by connexins.

### BACKGROUND

Connexins are intrinsic membrane proteins that form hexagonal arrangements in the plasmatic membrane called connexons or hemichannels. Two hemichannels may be bound by their extracellular segments in order to form channel gap junctions or gap junctions, which allow the direct transfer of several substances between cells (Sáez, Merthoud et al. 2003). In the last decade it has been shown that under physiological conditions, the hemichannels play an important role in the transfer of metabolites and nutrients (Chandrasekhar and Bera 2012) and also in the release of intercellular signaling molecules, such as ATP, glutamate, prostaglandin E2 and NAD⁺ providing a way in the membrane for the autocrine and paracrine signaling (Wang, De Bock et al. 2012). However, under different pathological conditions, the activity of the hemichannels formed by connexins is regulated upwards and significantly contributes to the result of the cellular degeneration (Sáez, Schalper et al. 2010).

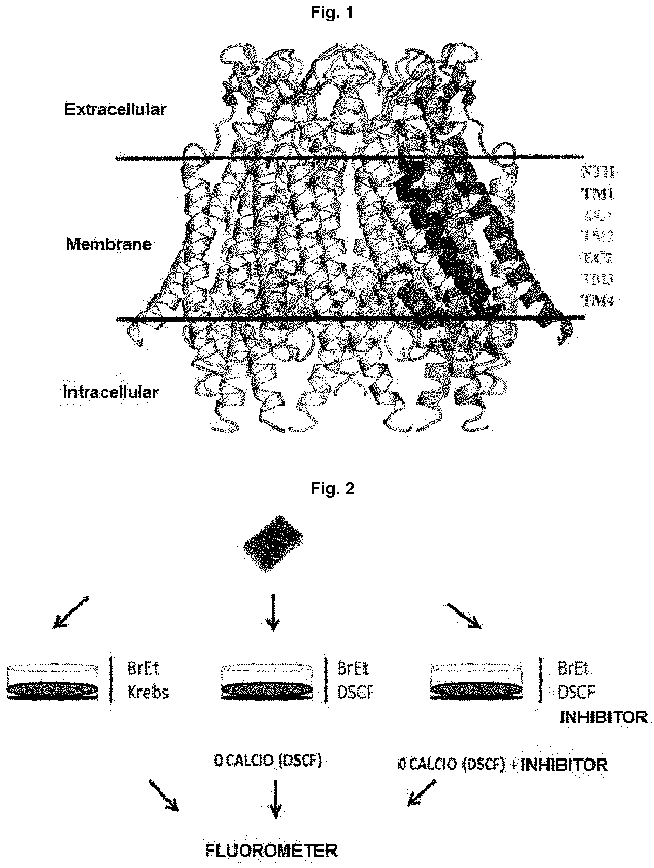

The recent determination of the crystalline structure of connexin 26 to 3.5Å of resolution through X ray crystallography (Maeda et al. 2009) shows that the hemichannels are the conserved motives of gap junctions and they can be considered as modules of the structural unit (Yeager, 2009). The hemichannel structure formed by connexin 26 shows that each monomer is made up by 4 transmembrane alpha helixes (TM1-TM4), 2 extracellular loops (E1, E2), 1 intracellular loop and one N-terminal helical domain (NTH) and the C-terminal domain. Each connection contains an arrangement of six protomers and each monomer of connexin 26 contains a typical four helix bundle, where any pair of adjacent helixes is antiparallel (Maeda and Tsukihara, 2011). The arrangement of the secondary structure of connexin 26 is as follows: TM1, TM2 and E1 face the pore; TM3, TM4 and E2 are located in the perimeter of the hemichannel in front of the membrane lipids or the extracellular environment, respectively; TM2 and the cytoplasmatic half of TM1 is covered by NTH and they are not exposed (Figure 1).

Several lines of research suggest that the use of specific inhibitors of channels formed by connexins can have a therapeutic usefulness in the treatment of epilepsy, cardiac arrhythmias, cancer, stroke or other conditions (Spray, Rozental et al. 2002). Although the use of electrophysiological techniques and in *in vitro* assays using recombinant proteins has allowed to identifying the drugs that allegedly act over hemichannels or channels to modulate their function directly, for most of, if not all, hemichannel inhibitors formed by connexins reported to date, the accurate mechanism of action through which their activity is modulated and their transport properties remain unknown (Juszczak and Swiergiel 2009). In addition, all compounds described to date are effective in micromolar concentrations, which shows that they are little selective and many of them also block the hemichannels formed by panexines (Varselis and Srinivas 2013). Therefore, pharmacological tools are urgently needed to further clarify their functions and validate them as pharmacological targets for the development of new therapies for diseases based on the dysfunction of connexins (Bodendiek and Raman 2010).

There are at present several connexin modulators, but most of them simultaneously affect the activity of hemichannels and channels formed by connexins. In addition, the use of antisense RNA or morpholinos that inhibit the expression of connexins eliminate the expression both of hemichannels and the gap junctions. Consequently, the compounds described in the art show little efficacy and unspecific mechanisms of action or secondary to the pharmacological action in other therapeutic targets that modify the activity of connexins (for ex. kinases).

In the state of the art there are for example patents or patent applications EP1469875, US7153822, WO2003063891, US20040092429, US20070042964 y WO2006134494 that describe compositions and methods to modulate the activity of hemichannels formed by connexins. Among the mechanisms proposed in such documents there is the phosphorylation of tyrosine residues in the C-terminal segment of connexin 43, blocking of the binding of 2 hemichannels by the use of peptidomimetic compounds that are similar to the bindig zone between hemichannels or transmembrane regions thereof that modify the formation of hemichannels. These compounds, however, are not selective for the transport through hemichannels and they also affect gap junction channels not being specific.

In addition, the patent application WO2007002139 claims a neuroprotection method in retinal cells by decreasing the release of connexin-mediated ATP in response to local hypertension. In this application it is specified that the hemichannel blockers can be mefloquine, meclofenamic acid, retinoic acid, 18-alpha-gliciretinic acid, flufenamic acid, riflumic acid, carbenoxolone, peptidomimetics or combinations thereof. However, these compounds are not selective for hemichannels formed by connexins, since they also block gap injunction channels and the pharmacological effect depends on the transport of ATP, which can be transported by alternative ways to hemichannels.

In other examples, such as application WO2009148613, the use of antisense oligonucleotides, interference RNA or siRNA is included to inhibit the expression both of the intercellular channels located between two cells, as the hemichannels located in the cellular surface. These oligonucleotides, however, are addressed to specifically modulate the connexin 43 isoform (in the human there are 20 further connexins) and they only allow formulation in pharmaceutical forms for transdermal, intravenous or deposit administration.

In the application JP2011506446, the use of antibodies or binding to antigens fragments is described, which are not useful in chronic inflammatory diseases, because strong rejection or allergic reactions could be generated. Due to this, the great interest for having drugs modulating the formation, opening or activities of the hemichannels forms by connexins is evident.
In the application US20110223204, the use of modulating agents is claimed, which are antisense oligonucleotides, ribozymes, RNAi or siRNA with lengths between 15-35 nucleotides and with at least 70% homology to the mRNA antisense sequence of connexin 43. These biological derivatives can be selected only by the isoform connexin 43 and its effect prevents both the formation of hemichannels and gap junction channels formed by connexin 43. The blocking of the formation of gap junctions is a detrimental side effect for the normal operation of organs expressing connexin 43. In addition, said compounds present the general limitations of the oligonucleotides, for example they cannot be administered orally and they should be formulated in another type of pharmaceutical forms of intravenous administration.

The document US20090143425 describes compounds deriving from quinolines affecting the gap junctions. However, these compounds are not selective inhibitors of hemichannels formed by connexins.

The application US20090163440 describes the modulation of ionic channels, in particular channels composed by connexin 43, where the drugs used for their modulation are selected from among different regulators of ionic channels known: amlodipine, bepridil, diltiazem, disopyramide, encainide, felodipine, gallopamil, isradipine, mexitil, nicardipine, nifedipine, nimodipine, nitrendipine, procainamide, quinidine, tetrodotoxin, verapamil. From all the options described above in the document, only quinidine shows activity over connexin channels, with this being unspecific and of low potency.

In the patent applications US20120135960 and US20110172188, the use of mefloquine is described, among others, as a connexin blocking agent. However, mefloquine is a non-selective blocker of the gap junction channels formed by connexins Cx26, Cx32, Cx36, Cx43, and Cx50. This compound does not selectively inhibit the hemichannels formed by said connexins.

Documents US20080131528, US20060228433 and US20080096854 describe the use of compositions that restore the functioning of gap junctions. The purpose of these compositions is to promote the activity of the gap junctions. However, no inhibiting effect of hemichannels formed by connexins is known for such compounds.
From that already described, it is derived that at present the blockers of hemichannels formed by the available connexins also inhibit the gap junctions that play an important part in the coordination of a number of electric and metabolic responses in the tissues. Therefore, obtaining blocking compounds specific for hemichannels is desirable that do not have an effect on the gap injunction channels, which could be useful to design therapeutic treatment in a rational way for different diseases, minimizing the undesired side effects.

As to the methodologies to identify new drugs, there are different approaches in the state of the art in the search of compounds of greater specificity. For example, the document US2014141099 shows drug-discovery methods, particularly inhibitors of the Aurora kinase and a pharmacophore that describes compounds that promote a conformational change in the protein Aurora B and which binding constant for the two-step process is Ki* and it also refers to compounds with the characteristics of the pharmacophore. Said method to select an inhibitor of Aurora B comprises the steps of determining Ki, the determination of Ki* and the selection of a compound with a preset Ki/Ki*. For the determination of Ki*, the compound under study is preincubated and the kinase Aurora; the test mixture is diluted and Ki* is determined for a period of time.

As to the search of compounds with a greater affinity to a certain receptor, the patent document US8131527 shows an identification method for a compound that is a modulator of the receptors of the fibroblast growth factor (FGF) with greater affinity to FGF than to VEGFR2, which comprises the steps of design and/or selection of a modulator using the pharmacophore represented by the given formula; then an in silico representation is generated of said modulator of the step (a); the atomic coordinates of a FGF protein are generated or a portion thereof for at least the binding pocket around Asp641; adjustment of one or more candidate modulators according to step (b) or one or more FGF residues in order to determine the probability that the candidate modulator can interact with FGF, where the NHs of the formula initially defined form a direct hydrogen bond with one or both Asp641 oxygens of the carboxylic acid; optionally, the modulator under study can be modified based on the results of the assembly stage; and putting the modulator under study in contact with FGF and/or VEGFR2 in order to determine the capacity of said candidate modulator of interacting with a preferred affinity for one of the receptors.

In addition, as to the patent application WO20004012683, it discloses a method to identify a therapeutic compound for a disease caused by an organism having a protein of SET domain which comprises analyzing the capacity of a compound under study to modulate the activity of at least one "druggable" region of the protein, where the modular capacity indicates a candidate therapeutic compound.

Furthermore, from such documents and the information available in the state of the art, it is derived that there are no backgrounds exploring effective and specific methodologies to identify and select compounds that are able of modulating the activity of hemichannels formed by connexins selectively.

### BRIEF DESCRIPTION OF THE FIGURES

Figure 1. Schematic representation of the basic structure of the connexin hemichannels. The secondary structure of a connexin hemichannel is composed of the hexameric arrangement of the connexin protomers. The domains of each protomer are indicated with names and coded by different intensities of grey and indicated by arrows. The membrane planes are represented by black lines.
Figure 2. Analysis scheme of the biologic assessment of the synthetic inhibitors of hemichannels where the Cx 43 cell cultures are subcultivated in 96-well black plates and subject to the 3 ethidium uptake analyses indicated to be assessed as fluorescence emitted in a fluorometer.
Figure 3. Ethidium uptake in HeLa Cx43-EGFP. Effect of DCFS. A: Cells in culture. B: Ethidium uptake chart in HeLa Cx43-EGFP. C: extracelular DCSF induces the inlet of ethidium in Cx43; the effect of DCSF is shown, which was blocked by the inhibiting molecule (MX) (10nM), (P<0,05, n=4).
Figure 4. Unit streams of the hemichannels formed by connexin 43 in HeLa cells bathed in saline solution without calcium ion (or Ca²⁺) and in the presence of EGTA.
Figure 5. Incidence percentage of coupling according to the time.
Figure 6. Cellular coupling assessed with the transfer of ethidium. A. Effect of the different inhibitors in HeLa-Cx43 cells (transfected with connexin 43). B. Cellular coupling index.
Figure 7. Functional status of the hemichannels formed by panexine1 (Panx1) driven by mechanic stress.

### DEFINITIONS

When the compounds, compositions and procedures of the invention are described, the following terms have the meanings indicated below, unless otherwise indicated.

The term "C₁₋₃ alkyl" refers to a hydrocarbonated chain comprising 1 to 3 carbon atoms.

The term "C₁₋₃ alkoxy" refers to a hydrocarbonated chain comprising 1 to 3 carbon atoms bound to an oxygen atom.

The term "therapeutically effective amount" refers to an amount enough to perform the treatment when administered to a patient in need of treatment.

The term "treatment", as used in this specification, refers to the treatment of a disease or pathologic medical condition in a human patient, including:
(a) Preventing the onset of a disease or pathologic medical condition, i.e. the prophylactic treatment of a patient;
(b) Relieving the disease or pathologic medical condition, i.e. causing the regression a disease or pathologic medical condition in a patient;
(c) Suppressing a disease or pathologic medical condition, i.e. delaying the development of the disease or pathologic medical condition in a patient, or
(d) Relieving the symptoms of a disease or pathologic medical condition in a patient.

The expression "disease or pathologic condition associated with activity of connexins" includes all the states of a disease and/or states currently recognized or to be found in the future, which are associated with an increase of the activity of hemichannels formed by connexins 26 and/or 43. Such states of disease or condition include, but they are not limited to, inflammatory diseases, vascular disorders, arrhythmias, chronic injuries, retinal neuroprotection, fibrosis, treatment of pain, skeletal muscle denervation, muscular dystrophies, damage to the spinal cord and genetic diseases characterized by the increased activity of hemichannels formed by connexins.

The term "pharmaceutically acceptable salt" refers to a salt prepared from a base or acid acceptable for administration to a patient, such as a mammal. Said salts can be obtained from inorganic or organic pharmaceutically acceptable bases and from inorganic or organic pharmaceutically acceptable acids.

Salts deriving from pharmaceutically acceptable acids include the following acids: acetic, benzenesulfonic, benzoic, canfosulfonic, citric, etanosulfonic, fumaric, gluconic, glutamic, bromhidric, clorhidric, lactic, maleic, malic, mandelic, metanosulfonic, mucic, niric, pantotenic, fosforic, succinic, sulphuric, tartaric, p-toluenesulfonic, xinafoic, (1-hidroxi-2-naftoic acid) and similar. The salts particularly preferred are those derived from fumaric, bromhidric, clorhidric, acetic, sulphuric, metanesesulfonic, xinafoic and tartaric acids.

Salts deriving from inorganic pharmaceutically acceptable include aluminum, ammonia, calcium, copper, ferric, ferrous, lithium, magnesium, manganic, manganese, potassium, sodium, zinc and similar salts. Particularly preferred are the ammonia, calcium, magnesium, potassium and sodium salts.

Salts deriving from pharmaceutically acceptable organic bases include salts of primary, secondary and tertiary amines, including amine substituted, cyclic amines, amines of natural origin and similar, such as arginine, betaine, caffeine, cholin N,N'-dibenzylethylenediamine, diethylamine, 2-diethylaminoetanol, 2-diethylamineetanol, etanolamine, ethylendiamine, N-ethylmorfolin, N-ethylpiperidine, glucosamine, histidine, isopropilamine, lysins, methylglucamine, morfoline, piperazine, piperidine, polyamine resins, procaine, purines, theobromine, triethylamine, trimethylamine, tripropylamine, trometamine and similar.

The term "solvate" refers to a complex or aggregate formed by one or more molecules of a solute, i.e. a compound of the invention or one of its pharmaceutically acceptable salts and one or more molecules of solvent. Said solvates are typically crystalline solids having a substantially fixed molar ratio of solute and solvent. Representative solvents include, as example, water, methanol, ethanol, isopropanol, acetic acid and similar. When the solvent is water, the solvate formed is a hydrate.

The term "or one of its pharmaceutically acceptable salts or solvate of its stereoisomers" includes all combinations of salts, solvates and stereoisomers of a compounds of the formula (A-G).

The term "druggable region" is used in reference to a nucleic acid, a polypeptide, complexes and similar, and it refers to a region of a connexin protein forming hemichannels, which is an objective or probable objective for binding an agent that reduces or inhibits the activity of the hemichannel.

For a polypeptide, a druggable region generally refers to a region where several aminoacids of a polypeptide would be able to interact with an agent. For a polypeptide or complex thereof, examples of the druggable regions are pockets and binding sites, interfaces between the domains of a polypeptide, grooves or surface complex or contours or surfaces of a polypeptide or complex able to participate in the interactions with other molecules, such as the cell membrane. In particular, a druggable region in the connexins will correspond to a pocket limited by the residues corresponding to segments of residues 3-10 (NTH), 29-40 (TM1), 74-93 (TM2) of a protomer and residues 29-40 (TM1) of an adjacent protomer in connexin 26 or the equivalent residues in other connexins.

### BRIEF DESCRIPTION OF THE INVENTION

The present invention refers to methods to identify specific and effective modulating compounds (blockers) of hemichannels formed by connexins, the compounds identified under this methodology and the pharmaceutical compositions comprising said compounds.

In an embodiment of the invention, procedures of use for such compounds are provided, as well as the preparation of useful medications to treat diseases associated with the increased activity of hemichannels formed by connexins, such as inflammatory diseases, vascular disorders, arrhythmias, chronic injuries, retinal neuroprotection, treatment of pain, skeletal muscle denervation, muscular dystrophies, damage to the spinal cord and genetic diseases characterized by the increased activity of hemichannels formed by connexins.

The use to treat inflammatory disorders in a subject comprises the administration to said subject of a therapeutically effective amount of a compound inhibiting the activity, opening or transport through a hemichannel formed by connexins.

### DETAILED DESCRIPTION OF THE INVENTION

In the present invention, a methodology to identify the drugs has been used on the basis of the three-dimensional structure of therapeutic target to solve the technical problem of obtaining effective and specific modulating compounds of hemichannels formed by connexins. The purpose of the present strategy of drug identification based on structures applied to connexins is obtaining new agents for modulating hemichannels formed by connexins with greater affinity and selectivity, which reduce side effects in the patients by being specific.

The biologic activity of the compounds described was checked through permeability tests of the cellular membrane and measurements of microscopic streams.
The method to identify specific inhibiting compounds of hemichannels formed by connexins according to the present invention starts with the analysis of the structure of the therapeutic target of different human connexins and from this to generate comparative models with other connexins. In order to prepare the protein for the molecular coupling, first the crystalline structure of the hemichannel formed by the human connexin 26 was completed.

The comparative models founded on the hypothesis that the binding of compounds to a pocket limited by the N-terminal helical (NTH) domain, the transmembrane segments of helixes TM1 and TM2 of a protomer and the transmembrane segments of helixes TM1 of the adjacent protomer prevents the conformational changes that are necessary to generate the opening/activation of the hemichannels formed by connexins.

A total of 500 models of hemichannels formed by the human connexins 26 or 43 were generated using the program Modeller v9.2 (Sali, 1995) and the best model selected according to the internal DOPE scoring was selected for further analysis and optimization. The validation of the structures was performed by using the tools available in the web server SAVES (http://nihserver.mbi.ucla.edu/SAVES).

Before the virtual screening process, the database of chemical compounds OpenNCI was filtered in order to eliminate the salts and counter-ions using the program FILTER v2.1 (OpenEye Scientific Software, Santa Fe, NM) and then converted to 3D coordinates using the program OMEGA v2.4.6 (OpenEye Scientific Software, Santa Fe, NM), thus providing a database of about 195,000 compounds and conformers. They were subject to a comprehensive molecular coupling protocol in the modified crystalline structure modified of the hemichannel formed by connexin 26 and the model of hemichannel formed by connexin 43, using the program FRED v2.2.5 (OpenEye Scientific Software, Santa Fe, NM). The binding sites of the hemichannel formed by connexin 26 and the model of hemichannel formed by connexin 43 were identified and prepared using the FRED receptor program.

A campaign of virtual screening is performed in order to identify potential modulators of hemichannels formed by connexin using the structure of the hemichannel composed of human connexin 26 and a hemichannel model formed by human connexin 43 developed by comparative modeling using the structure of human connexin 26 as pattern.

The candidate binding modes of ligands at the receptor site (100) were obtained optimized by using the Chemgauss3 scoring function. The structures of consensus for the binding modes of the comprehensive molecular coupling process and optimization were obtained through the consensus scoring using the assessment functions PLP, Chemscore and Chemgauss3. Finally, the binding modes for the best classified 1,000 compounds were minimized with the force field CHARMm22 en DiscoveryStudio v2.1 (Accelrys Inc., San Diego). The protocol allows minimizing the side chains of residues inside 6Å from the mass centroid of all coupled ligands, using the conjugated gradient algorithm up to a convergence criterion of 0,001 Å kcal/mol for the root mean square (RMS) of the energy gradient.

The evaluation of the binding energy was performed for each complex obtained using the evaluation functions PLP, LigScore, PMF and LUDI. For the final identification of compounds with inhibiting potential, the consensus scoring protocol available in Discovery Studio v2.1 was used. From this new classification, the results were visually inspected for the compounds with the 100 best results and 40 compounds were selected within the group showing the best affinity to hemichannels formed by connexin 26 or connexin 43.

The compounds are tested in experiments based on cells that are deficient in the expression of hemichannels and transfected with different connexins in order to evaluate and/or confirm their capacity of modulating the transport through the cellular membrane. The transport of permeability tracing molecules is evaluated and compared with the inhibitors of channels formed by connexins known, such as beta glycyrrhetinic acid (BGA) and carbenoxolone (CBX).

The cells are incubated in an extracellular medium without divalent cations in order to increase the opening probability of hemichannels formed by connexins. In addition, as negative control parental cells not expressing hemichannels and cells transfected with panexine 1 and mechanically stimulated to induce the opening of the hemichannels formed by panexine 1.
The molecules that inhibit the hemichannels formed by connexins are evaluated by their action on the hemichannels formed by panexine 1. The molecules inhibiting the hemichannels formed by connexins in the nanomolar range are studied as possible inhibitors of intercellular binding channels using the coupling technique to dyes and through electrophysiology using double fixing of voltage.

Should the molecules not inhibit the intercellular channels formed by connexins in the millimolar range and not affect the hemichannels formed by panexine 1, its inhibiting effect of currents of hemichannels formed by connexins using the voltage fixing methodology in the modality of full cell.

The invention provides new inhibiting compounds of hemichannels formed by the connexins 26 and/or 43.

An objective of the present invention is to provide a method to identify compounds with inhibiting potential over the hemichannels formed by connexins, where a virtual screening method of ligands is used over a crystallographic structure or a comparative model of proteins corresponding to a hemichannel formed by connexin 26 or connexin 43. The method characterizes for evaluating the interaction energy of compounds at a binding site by the residues corresponding to the segments of residues 3-10 (NTH), 29-40 (TM1), 74-93 (TM2) of a protomer and the residues 29-40 (TM1) of an adjacent protomer in connexin 26 or equivalent residues in other connexins.

The method of the present invention allows determining that the compounds identified inhibit the opening/activation of hemichannels formed by connexins induced by the absence of divalent cations in the extracellular medium or by the application of positive voltages to the cellular membrane, thus avoiding dependence on the mechanism that induces the opening of the hemichannels.

Another objective of the invention is to provide a pharmaceutical composition comprising the compounds identified in a therapeutically effective amount of one or more specific modulators of hemichannels formed by connexins according to the present invention accessing the action site in the cells to be treated. The pharmaceutical compositions comprising at least a compound of the present invention optionally comprise at least one pharmaceutically acceptable vehicle.

Another objective of the invention is the use of one or more modulators of hemichannels formed by connexins in the preparation of a medication for the treatment and/or prevention of diseases associated with the over-activation of hemichannels formed by connexin 26 or connexin 43, where said modulators of hemichannels formed by connexins reach the action site in said cells.

The compounds obtained under the present invention can be used to prepare a useful medication for the prevention and/or treatment of inflammatory diseases, vascular disorders, arrhythmias, chronic injuries, retinal neuroprotection, treatment of pain, skeletal muscle denervation, muscular dystrophies, damage to the spinal cord and genetic diseases characterized by the increased activity of hemichannels formed by connexins, where said compositions comprise therapeutically effective amounts of one or more agents that inhibit the opening/activation of hemichannels formed by connexins in the cells to be treated.

The compounds identified through the method according to the present invention only blocked hemichannels and not intracellular channels formed by connexins or hemichannels formed by panexine 1. The new inhibiting compounds of hemichannels formed by connexins directly bind to the hemichannels and show better efficacy and strength than the compounds described in the state of the art.

In short, the method to obtain specific inhibiting compounds of connexin hemichannels according to the present invention comprises the steps of:
- Generate and validate comparative models of hemichannels formed by the connexins in question;
- Analyze the structure and define the interaction site of the connexin in reference;
- Generate a database of chemical compounds in a three-dimensional format containing the partial loads and the optimal geometry of each compound, eliminating salts and counter-ions;
- Perform a virtual screening in order to identify modulators of hemichannels formed by connexins;
- Select blocking compounds of the hemichannels formed by connexins based on a function of energy allowing to estimating the relative affinity of each compound to the connexin in reference; evaluate the capacity of the compounds of modulating the transport through the cellular membrane in cells being deficient in the expression of hemichannels, transfected with different connexins as to the inhibiting compounds in reference;
- Determine that the compounds identified inhibit the opening/activation of hemichannels formed by connexins induced by the absence of divalent cations in the extracellular mean or by the application of positive voltages to the cellular membrane; and
- Classify the molecules that inhibited the hemichannels formed by connexins according to their action over the hemichannels.

### PHARMACEUTICAL COMPOSITIONS

The pharmaceutical formulations can be conveniently presented in the form of unit dose and they can be prepared through any of the procedures well known in the pharmaceutical art. All procedures include the step of putting the active ingredient or ingredients in contact with the vehicle. In general, the formulations are prepared putting the active ingredient in contact with liquid vehicles or solid vehicles finely divided in a uniform way, and then, if necessary, forming the product under the formulation desired.

The proper formulations of the present invention for oral administration can be presented as capsules or tablets, with each of them containing a preset amount of active ingredient, as a powder or granule; as a solution or suspension in an aqueous liquid or in a non-aqueous liquid; or as oil in water liquid emulsion or water in oil liquid emulsion. The active ingredient can be also present in the form of injection, medicinal powder mixed with honey or syrup or thick ointment. A syrup formulation will in general consist in a suspension or solution of the compound or salt in a liquid vehicle.

The typical dermal and transdermal formulations comprise a conventional aqueous or non aqueous vehicle, as for example cream, ointment, lotion or thick ointment or they are in the form of medicated plaster, patch or membrane.

### EXAMPLES

### Example 1:

*In vitro* test of blocking the opening/activation of hemichannels formed by connexins (26 or 43). The capacity of the different tracing molecules was tested *in vitro* to evaluate the capacity of the compounds of reducing the transport of these molecules using the HeLa cells transfected with connexins 26 or 43.

**Table 1: Inhibition percentage to the permeability tracer at a concentration of 5µM.**

| | % Inhibition |
|---|---|
| Compound | Hemichannel activity (Cx26 o 43) |
| Example 1 (A) | 0 |
| Example 2 (B) | 20 |
| Example 3 (C) | 0 |
| Example 4 (D) | 100 |
| Example 5 (E) | 100 |
| Example 6 (F) | 0 |
| Example 7 (G) | 0 |
| Control (CBX, ABG) | 100 |

The *in vitro* most potent inhibitor was example 4 with IC₅₀ of 20 nM and 100% efficacy, followed by example 5 with IC₅₀ of 50 µM and 100% efficacy.

As reference, carbenoxolone or glycyrrhetinic acid (CBX, ABG) was used as control, which is a blocker of connexins widely used that showed an IC₅₀ of 100 µM and 100% efficacy.

Therefore, the inhibitors of the present invention show a proper blocking activity of the opening/activation of the hemichannels of connexin 26 and 43 *in vitro.*

### Example 2:

Use of a blocking compound of the activity of the hemichannels formed by connexin 26 or connexin 43.

The method comprises putting in contact selectively or specifically a hemichannel formed by connexins 26 or 43 with a therapeutical effective amount (in the nanomolar range) of at least two of the following compounds:
A:(*R*)-2-(4-chlorofenil)-2-oxo-1-fenilethylquinoline-2-carboxylate,
B:1,3-bis(4-(4-chlorofenil)piperazin-1-il)propane
C: Acetic 2,2-bis([1,1'-bifenil]-4-iloxi) acid.
D:(2*R,*5*S,*8*R*,9*S*,10*S*,13*S*,14*S*,17*S*)-2-fluoro-10,13-dimethyl-3-oxohexadecahydro-1*H-*ciclopenta[a]fenantren-17-il benzoate.
E:(3S,5S,8R,9S,10S,13S,14S,17S)-3-acetoxi-10,13-dimethylhexadecahydro-1H-ciclopenta[a]fenantren-17-il ciclohexanocarboxilate.
F:(3S,8R,9S,10R,13S,14S,17R)-17-ethynil-17-hydroxi-10,13-dimethyl-2,3,4,7,8,9,10,11,12,13,14,15,16,17-tetradecahydro-1H-ciclopenta[a]fenantren-3-yl 3-ciclohexilpropanoate.
G: bis(4-methyl-2-morfolinquinoline-6-yl)methane.

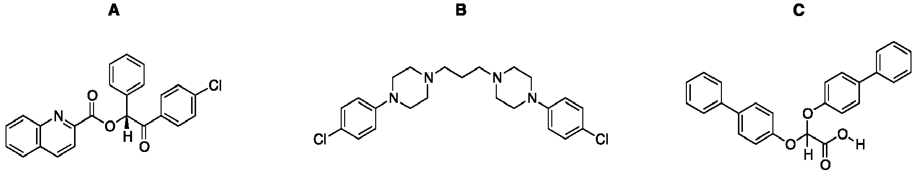

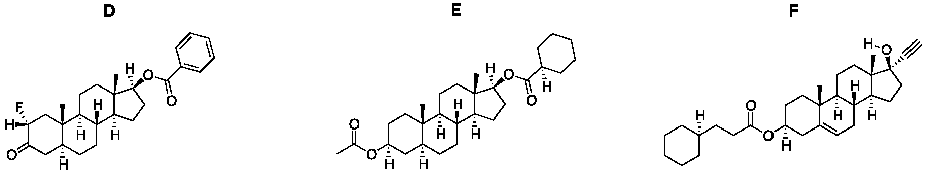

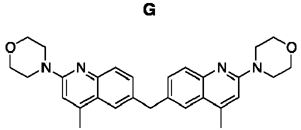

The best results are obtained for A and D, because they became potent inhibitors of hemichannels formed by connexins and they do not affect the gap junction channels or hemichannels formed by panexines.

### Example 3: Biologic evaluation

Mouse HeLa cells transfected with connexins 26 or 43 are used. In order to evaluate the activity of each compound over the connexin hemichannels, 10³ HeLa cells transfected with connexins are sown by well in multi-well (90) plates 24 h before each experiment. Then, the cells are washed in a Locke solution that contains normal levels of divalent cations (Ca²⁺ and Mg²⁺) or a cation-free divalent Locke solution that contains 5 µm of ethidium bromide. Parallel to this, the cells are treated with different concentrations of each candidate compound and incubated for 5 min.

As indicated by the scheme of Figure 2, the HeLa cells transfected with connexin 43 (Cx43) are sown in multi-well (96) plates and after 24 h the ethidium uptake is evaluated in the presence of cation free solution (DCFS) in the presence or absence of the compound in question. The ethidium uptake is evaluated as the fluorescence emitted measured with a fluorometer. If the value of fluorescence in the presence of the inhibitor is equal or close to that measured in cells bathed with a solution that contains divalent cations, the compound corresponds to an inhibitor of hemichannels formed by connexins. On the contrary, if the fluorescence value emitted by cells exposed to a possible inhibitor in DCFS is equal to that of cells bathed only with DCFS (without the compound in question), the compound is not an inhibitor of hemichannels formed by connexins. The same test is performed with different concentrations of each compound in order to determine the minimum effective concentration (EC₅₀).

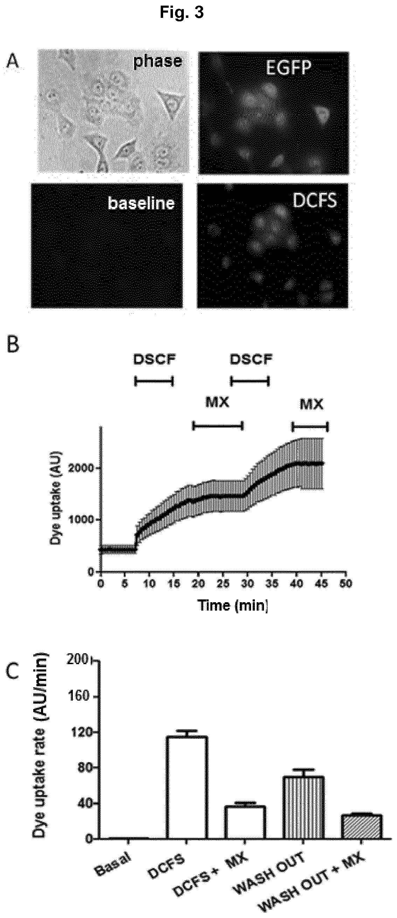

In Figure 3, the upper left panel (A) corresponds to a phase microphotograph, where the HeLa cells can be noted that express connexin 43 conjugated with green fluorescent protein (EGFP) present in the microscopic field. On the right side, fluorescence is depicted indicating that all cells of the field express connexin 43. The bottom left panel shows the uptake of ethidium at baseline conditions (presence of Ca²⁺ in the extracellular medium) and the bottom right panel shows the ethidium uptake 5 minutes after the extracellular solution has been changed by one without divalent cations (DCFS), which increases the activity of the hemichannels. The chart (B) shows the activity of the hemichannels as "Dye uptake" in arbitrary units, AU, in at least 40 cells bathed in the control medium followed by DCFS, the application of 10 nM of inhibiting molecule (R)-2-(4-chlorofenil)-2-oxo-1-fenilethlyquinoline-2-caroxilate (compound 4 or inhibiting molecule), washed with DCFS and followed by a new application of 10 nM of inhibiting molecule (MX). Clearly, the inhibiting molecule inhibits the activity of hemichannels evaluated as dye uptake. Panel (C) shows that the ethidium uptake speed or rate by minute is completely inhibited by the inhibiting molecule and its effect is partially reversed with the washing of DCFS.

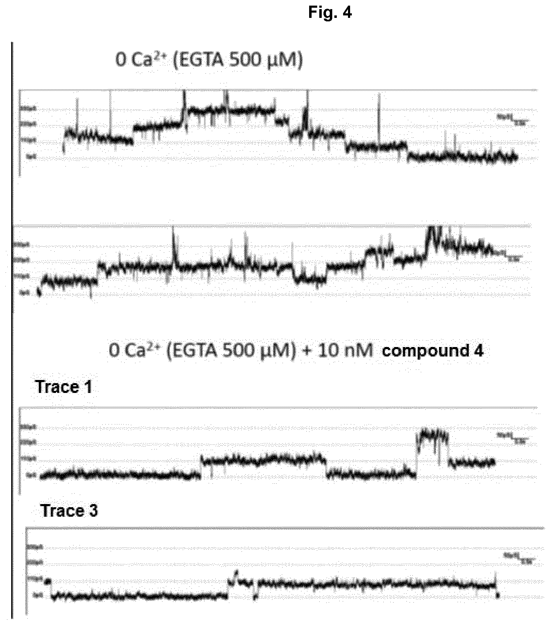

The unit currents of the hemichannels formed by connexin 43 are very frequent in the HeLa cells bathed with a saline solution without the calcium ion (or Ca²⁺) (see Figure 4, the two upper tracings). However, the application of 10 nM of compound 4 (inhibiting molecule) drastically inhibited the activity of the hemichannels formed by connexin 43 (the two bottom tracings 1 and 3).

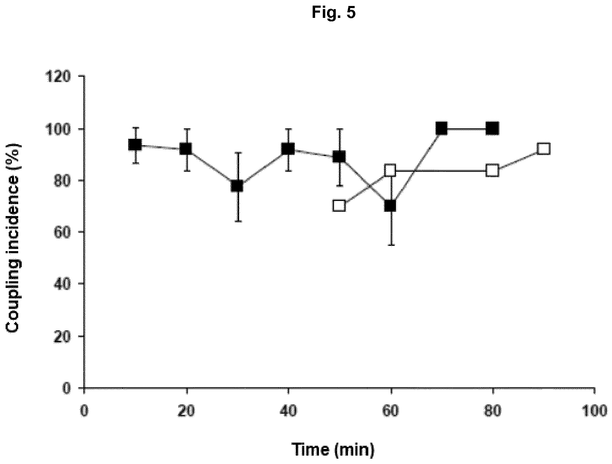

Figure 5 depicts that the cells transfected with connexin 26 are very well coupled to gap junction channels. The incidence of the cellular coupling evaluated with microinjections of Lucifer yellow is close to 100% (black boxes). It also shows that the addition of 1 nM of compound 4 does not significantly reduce the coupling incidence (white boxes), indicating that the functional status of the gap injunction channels formed by connexin 26 is not affected.

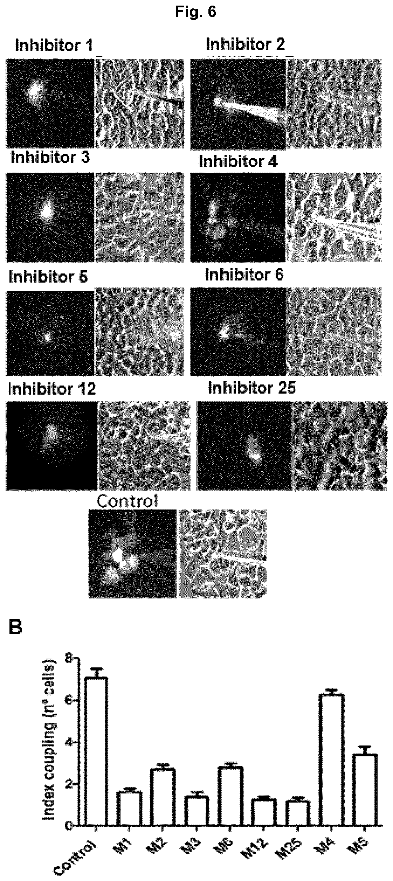

Figure 6 depicts that the application of 1 nM compound 4 (inhibiting molecule) does not inhibit the cellular coupling mediated with gap injunction channels, while other compounds evaluated inhibit the gap junction channels formed by connexin 43 totally or in part.

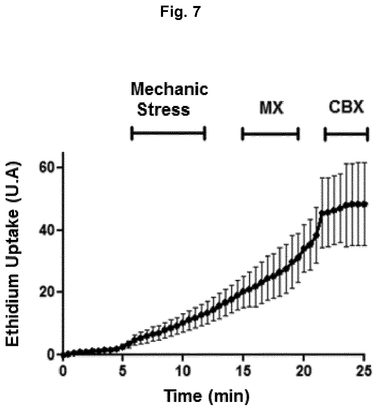

The functional status of the hemichannels formed by panexine 1 (Panx1, Figure 7) activated by mechanic stress is not inhibited by the application of 100 nM of compound 4, but it is totally blocked by the application of 5 µm of carbenoxolone. This result indicates that concentrations of the nanomolar range of compound 4 inhibit the hemichannels formed by connexin and not those formed by panexine 1 and that also the compound does not inhibit the gap junction channels formed by connexin. Therefore, the compound is an inhibitor of hemichannels formed by highly specific conexins.

### REFERENCES

Bodendiek, S. B. and G. Raman (2010). "Connexin modulators and their potential targets under the magnifying glass." Curr Med Chem 17(34): 4191-4230.
Chandrasekhar, A. and A. K. Bera (2012). "Hemichannels: permeants and their effect on development, physiology and death." Cell Biochemistry and Function 30(2): 89-100.
Juszczak, G. R. and A. H. Swiergiel (2009). "Properties of gap junction blockers and their behavioural, cognitive and electrophysiological effects: animal and human studies." Prog Neuropsychopharmacol Biol Psychiatry 33(2): 181-198.
Saez, J. C., V. M. Berthoud, M. C. Branes, A. D. Martinez and E. C. Beyer (2003). "Plasma membrane channels formed by connexins: their regulation and functions." Physiol Rev 83(4): 1359-1400.
Saez, J. C., K. A. Schalper, M. A. Retamal, J. A. Orellana, K. F. Shoji and M. V. Bennett (2010). "Cell membrane permeabilization via connexin hemichannels in living and dying cells." Exp Cell Res 316(15): 2377-2389.
Spray, D. C., R. Rozental and M. Srinivas (2002). "Prospects for rational development of pharmacological gap junction channel blockers." Curr Drug Targets 3(6): 455-464.
Verselis, V. K. and M. Srinivas (2013). "Connexin channel modulators and their mechanisms of action." Neuropharmacology 75(0): 517-524.
Wang, N., M. De Bock, E. Decrock, M. Bol, A. Gadicherla, M. Vinken, V. Rogiers, F. F. Bukauskas, G. Bultynck and L. Leybaert (2012). "Paracrine signaling through plasma membrane hemichannels." Biochim Biophys Acta.

## Claims

1. A method to identify specific inhibiting compounds of connexin hemichannels, WHEREIN it comprises:
a) alyze the structure of the connexin in reference;
b) Generate comparative models of connexins;
c) Perform a virtual screening in order to identify modulators of hemichannels formed by connexins;
d) Evaluate the capacity of the compounds of modulating the transport through the cellular membrane in cells being deficient in the expression of hemichannels, transfected with different connexins as to the inhibiting compounds in reference;
e) Classify the molecules that inhibited the hemichannels formed by connexins according to their action over the hemichannels.

2. A method according to claim 1, wherein the connexins of reference correspond to connexins 26 and/or 43.

3. A method according to claim 1, wherein in the step (a) the connexin of reference is the human connexin 26.

4. A method according to claim 1, wherein in step (b) the comparative model is based on the evaluation of the interaction energy in the domain that prevents the conformational changes in order to generate the opening/activation of the hemichannels formed by connexin.

5. A method according to claim 4, wherein the interaction energy is evaluated in the domain defined by the N-terminal helical (NTH) and the transmembrane segments of alpha-helixes 1 and 2 of a connexin protomer and the transmembrane segment of the alpha-helix 1 of another protomer in a hemichannel.

6. A method according to claim 1, wherein in the step (c) the virtual screening is performed using the structure of hemichannel composed of the connexin of reference and a hemichannel model formed by a comparison connexin through comparative modeling using as pattern the structure of the connexin of reference.

7. A method according to claim 6, wherein in the step (c) the comparison connexin is connexin 43.

8. A method according to claim 6, wherein in the step (c), the virtual screening of ligands used is over a crystallographic structure or a comparative model of proteins corresponding to a hemichannel of connexin 26, 43 or other.

9. A method according to claim 8, wherein the interaction energy of the compounds is evaluated in a binding site limited by the residues corresponding to the segments of residues 3-10 (NTH), 29-40 (TM1), 74-93 (TM2) of a protomer and residues 29-40 (TM1) of an adjacent protomere in connexin 26 or the equivalent residues in other connexins.

10. A method according to claim 1, wherein in step (d) the reference inhibitors are the beta glycyrrhitenic acid (BGA), carbenoxolone (CBX) or mixtures thereof.

11. A method according to claim 1, wherein in step (d) the evaluation is performed using cells incubated in an extracellular medium without divalent cations.

12. A method according to claim 1, wherein in step (d) a negative control of parental cells not expressing connexin hemichannels was used.

13. A method according to claim 1, wherein in step (d) cells transfected wit panexine 1 are used as control.

14. A method according to claim 13, wherein in step (d), cells are mechanically stimulated in order to induce the opening of hemichannels formed by panexine 1.

15. A method according to claim 1, wherein in step (d) cells are put in contact with at least one compound selected in the binding site limited by the residues corresponding to segments of residues 3-10 (NTH), 29-40 (TM1), 74-93 (TM2) of a protomer and residues 29-40 (TM1) of an adjacent protomer in connexin 26 or the equivalent residues in other connexins and where a change in permeability of hemichannels of Cx26 or Cx43 to the tracer is detected, where the change in the permeability of the tracer in indicative of the modulation of the hemichannel by a compound.

16. Use of specific inhibiting compounds of connexin hemichannels obtained according to the method of claim 1, wherein they are useful for the preparation of a medication for the treatment and/or prevention of diseases associated with the over-activation of hemichannels of connexin 26 or 43.

17. Use of specific inhibiting compounds of connexin hemichannels obtained according to the method of claim 1, wherein they are useful for the preparation of a medication for the treatment and/or prevention of inflammatory diseases, vascular disorders, arrhythmias, chronic injuries, retinal neuroprotection, fibrosis, treatment of pain.

18. Use according to claims 16 or 17, wherein the medication contains one or more specific inhibitors of hemichannels of connexin selected from quinoline derivatives or cyclopentafenantren or combinations thereof.

19. Use according to claim 18, wherein the quinoline derivative is (R)-2-(4-chlorofenil)-2-oxo-1-fenilethylquinoline-2-carboxylate.

20. Use according to claim 18, wherein the cyclopentafenantren derivative is (2*R*,5*S*,8*R*,9*S*,10*S*,13*S*,14*S*,17*S*)-2-fluoro-10,13-dimethyl-3-oxohexadecahydro-1*H* ciclopenta[*a*]fenantren-17-il benzoate.
